# EUROPEAN PATENT APPLICATION

(11) **EP 4 495 976 A1**
(43) Date of publication of application: **22.01.2025**
(21) Application number: 22931343.2
(22) Date of filing: 16.03.2022
(51) Int. Cl.: H01J 49/02, H01J 49/04, H01J 49/16, H01J 49/00, G01N 27/62

(54) **DEVICE FOR ONLINE RAPID PRETREATMENT MASS SPECTROMETRY OF MULTI-CELL OR SINGLE-CELL SAMPLE AND APPLICATION THEREOF**

(71) Applicant: University of Science and Technology of China, Anhui 230026 (CN)
(72) Inventor: HUANG, Guangming, Hefei, Anhui 230026 (CN); HOU, Zhuanghao, Hefei, Anhui 230026 (CN)
(74) Representative: Altmann Stößel Dick Patentanwälte PartG mbB
(86) International application number: PCT/CN2022/081091
(87) International publication number: WO 2023/173305

(57) **Abstract**

The present disclosure provides an integrated device for rapid pretreatment-ionization-detection comprising: (a) a fixing device for fixing a sample to be treated; b) a rapid pretreatment-ionization device comprising: a voltage output module for generating an AC voltage; a boost module for boosting and amplifying the voltage to achieve an adjustable pulse voltage of 1mV-100 kV; a delay module for setting a delay time for the voltage output; and a trigger module for receiving a pulse voltage trigger; (c) a mass spectrometer; and (d) a timing control device for controlling the mass spectrometer and the rapid pretreatment-ionization device to collect a signal. According to the integrated device, mutual separation between analytes in a cell sample and separation between an analyte and a complex matrix can be achieved, and the separation time can reach a sub-millisecond level.

## Description

### FIELD OF THE INVENTION

The present disclosure relates to the field of biological mass spectrometry analysis, and in particular to a device for online rapid pretreatment mass spectrometry analysis of biological molecules in multi-cell or single-cell samples, and more particularly to an integrated device for rapid pretreatment-ionization-detection, which is used for online rapid pretreatment mass spectrometry analysis of multi-cell or single-cell samples.

### BACKGROUND OF THE INVENTION

Cells are the basic units that constitute organisms. They have different phenotypes, genotypes and epigenetic states in living organisms. In the process of development, differentiation, aging and apoptosis, accompanied by the rapid metabolism of various components in cells, there is a wide range of heterogeneity. Cell heterogeneity is mainly reflected in three aspects: nucleic acid, protein and metabolism. There are differences between different cells in gene mutation, recombination and epigenetic modification. There are also differences in the types and activities of transcriptional regulation between different types of cells during transcription, which brings about cell heterogeneity at the nucleic acid level. At the same time, there are differences in the expression of various proteins in cells and the degree of post-translational modification, and there are also individual differences in the regulation of cell signaling pathways, resulting in significant heterogeneity of cells at the protein level. The difference in the state of signal pathways in cells will directly affect the difference in metabolic levels in cells. In addition, the different environments of the cells will result in different levels of cell uptake of external nutrients, which leads to significant cell heterogeneity at the metabolic level.

In previous research strategies, homogenization is usually used, which could only obtain the overall signal level in tissues and organs at the group level. However, studying the molecular mechanisms of cells in the process of life activities at the single-cell level can more deeply study and analyze the fine composition of cells inside tissues and organs, understand the fine life regulation mechanisms of cells in physiological or pathological processes, and find and discover cell subpopulations that play important functions in tissues and organs. It has important biological research significance and can provide new perspectives for the detection of circulating tumor cells, the discovery of potential biomarkers related to diseases, and personalized diagnosis and treatment.

The multi-cell/single-cell strategy based on electrospray can ionize biological molecules simultaneously, and the sensitivity of response is high. It is suitable for the analysis and detection of multiple biological molecules in multi-cell/single-cell, and is an important analytical tool for multi-cell/single-cell analysis.

However, due to the small size of cell samples, which is of only picoliters, and the many types of biomolecules in multi-cell/single-cell samples, the wide range of concentrations in which they function, and the presence of a large number of inorganic salt interferences, direct sampling and ionizing will result in severe ion suppression, which often only allows the analysis of high-abundance biomolecules in cells, such as lipids, choline and other substances with quaternary ammonium groups in cancer cells, and oligosaccharides in plants. It is difficult to achieve the coverage of low-abundance biomolecules with important functions in multi-cell/single-cell samples.

For complex single-cell environments, offline or online separation and removal of matrix can effectively reduce the interference of a complex matrix on cells, but the pretreatment speed is slow and dilution to nanoliters or microliters is required. The dilution effect leads to a decrease in analytical sensitivity, and long-term pretreatment is prone to cause degradation and in vitro transformation of biomolecules, making it difficult to ensure the detection of biomolecules under physiological conditions.

Rapid pretreatment separation, improving coverage of biomolecules and their pathways, and avoidance of degradation and transformation of biomolecules in multi-cell/single-cell samples pose new challenges to mass spectrometry.

### SUMMARY OF THE INVENTION

The present disclosure provides a metabolic mass spectrometry analysis method for a multi-cell/single-cell sample, and the invention is characterized in providing "an integrated device for rapid pretreatment-ionization-detection" for sub-millisecond pretreatment of samples, which is different from the prior art in that the present disclosure provides "an integrated device for rapid pretreatment-ionization-detection" which achieves the separation between different analytes, and the separation between analytes and a complex matrix in a cell sample. The separation time reaches a sub-millisecond level, while the separation time of the prior art is usually several minutes to tens of minutes.

Specifically, the present disclosure provides the following technical solutions.

On the one hand, the present disclosure provides an integrated device for rapid pretreatment-ionization-detection, wherein the device comprises:
(a) a fixing device for fixing the sample to be treated, wherein the fixing device comprises a support frame for supporting a sample and a support frame for electrode for pretreatment and ionization; the support frame for supporting a sample fixes the sample to be treated by a snap-on or flat-lay contact; the electrode for pretreatment and ionization is a non-contact electrode or a contact electrode, and is used to apply voltage to the sample to be treated;
(b) a rapid pretreatment-ionization device, wherein the rapid pretreatment-ionization device comprises: a voltage output module for generating an AC voltage; a boost module for boosting and amplifying the voltage to achieve an adjustable pulse voltage of 1mV-100 kV; a delay module for setting a delay time for the voltage output; a trigger module for receiving a pulse voltage trigger;
(c) a mass spectrometer; and
(d) a timing control device for controlling the mass spectrometer to collect a signal and the rapid pretreatment-ionization device, wherein the mode of the timing control device is selected from:
   (1) the mass spectrometer outputs a trigger signal to the timing control device at an adjustable frequency of 1Hz-1M Hz, and the timing control device sends an adjustable 1mV-30V high-level signal to the rapid pretreatment-ionization device;
   (2) during sample pretreatment, the rapid pretreatment-ionization device outputs a trigger signal to the timing control device at an adjustable frequency of 1Hz-1M Hz, the timing control device sends an adjustable 1mV-30V high-level signal to the mass spectrometer, and the mass spectrometer receives the trigger signal to perform ion injection analysis;
   (3) the timing control device outputs a trigger signal at an adjustable frequency of 1Hz-1M Hz, outputs an adjustable 1mV-30V high-level signal to control the rapid pretreatment-ionization device to perform ionization, and triggers the mass spectrometer to collect a signal;
   (4) the timing control device monitors the mass spectrometry signal at an adjustable frequency of 1Hz-1M Hz, and after capturing the mass spectrometry signal, it outputs an adjustable 1mV-30V high-level signal to the rapid pretreatment-ionization device; by switching the detection mode of the mass spectrometer, the detection of a positive ion signal and a negative ion signal is achieved.

In some embodiments, a snap-on/flat-lay fixation is used to achieve firm fixation and rapid switching of samples.

In some embodiments, a non-contact electrode is used to avoid contamination of samples by electrodes and improve the reliability of analysis.

In some embodiments, the AC voltage output module and the boost module can achieve sub-millisecond rapid separation and pretreatment of samples.

In some embodiments, the trigger module can start and stop pretreatment, which can greatly improve the signal strength of the sample.

In some embodiments, the delay module can achieve the precise entry of pretreated ions into the mass spectrometer.

In some embodiments, the mass spectrometer is, for example, but not limited to, a magnetic mass spectrometer, a quadrupole mass spectrometer, an ion trap mass spectrometer, an orbital trap mass spectrometer, a time-of-flight mass spectrometer, and a Fourier transform ion cyclotron resonance mass spectrometer.

In some embodiments, the mass spectrometer is selected from a magnetic mass spectrometer, a quadrupole mass spectrometer, an ion trap mass spectrometer, an orbital trap mass spectrometer, a time-of-flight mass spectrometer, and a Fourier transform ion cyclotron resonance mass spectrometer.

In some embodiments, the four modes of timing control can achieve precise matching of the pretreatment process and the mass spectrometry analysis process, improve the collection effect of the pretreatment results, and achieve sub-millisecond monitoring of the sample separation.

In some embodiments, the AC voltage output by the voltage output module is not limited to a sine wave, a pulse wave, or a square wave.

In some embodiments, the AC voltage output by the voltage output module is a sine wave, a pulse wave, or a square wave.

In some embodiments, the frequency of the pulse voltage achieved by the boost module is adjustable in the range of 1m Hz-100 kHz, and the number of pulse cycle output is 1-100 k cycles.

In some embodiments, the delay time is 1ns-100ks.

In some embodiments, the pulse voltage trigger received by the trigger module is positive polarity 1mV-5V or negative polarity 1mV-5V to realize ion signal detection in mass spectrometry positive ion mode and negative ion mode.

In some embodiments, the mode of the pulse voltage trigger received by the trigger module is selected from edge trigger, pulse width trigger, slope trigger and duration trigger.

In some embodiments, the trigger source of the pulse voltage trigger received by the trigger module is selected from internal trigger, external trigger and power supply trigger.

In some embodiments, the pulse cycle of the pulse voltage trigger received by the trigger module is an AC cycle of rapid pretreatment with a speed consistent with the pulse width of pulse cycle; by connecting a capacitor and a high-voltage rectifier in front of the electrode, the AC high voltage is converted into a DC high voltage.

In some embodiments, the conversion of AC high voltage into DC high voltage, combined with a pretreatment device, can realize the monitoring of the precise separation at the initial spraying of DC electrospray.

In some embodiments, the fixing device is connected to the rapid pretreatment-ionization device by a high voltage line; the rapid pretreatment-ionization device and the timing control device are connected by e.g., a BNC interface, but are not limited to such an interface, and the timing control device and the mass spectrometer are connected by e.g., a BNC interface, but are not limited to such an interface.

In some embodiments, the fixing device is connected to the rapid pretreatment-ionization device by a high voltage line, the rapid pretreatment-ionization device and the timing control device are connected by a BNC interface, and the timing control device and the mass spectrometer are connected by a BNC interface.

On the other hand, the present disclosure provides a method for mass spectrometry analysis of a multi-cell/single-cell sample, wherein the method comprises the following steps:
Step (1), a standard solution is filterd to remove fine particles for later use; multi-cell samples such as tissue blocks and bacterial liquids are crushed to form a homogenate, or the samples are dispersed to prepare a cell suspension; experimentally synthesized samples or environmental samples containing a complex matrix, such as animal, plant, soil, river water and sediment samples, are prepared into a solution suspension or supernatant after centrifugation;
Step (2), samples containing a single cell are subjected to single cell extraction, culture and dispersion to obtain a single cell suspension or a single cell monolayer slide;
Step (3), the homogenate or cell suspension obtained in step (1) is sampled by direct syringe extraction or sampling under a microscope with a sampling volume of, for example, 0.001 pL-10 mL, placed on an integrated device for rapid pretreatment-ionization-detection, wherein the integrated device performs online pretreatment, ionization and mass analysis of the sample to obtain relevant information of a substance in the sample;
Step (4), the single cell sample in step (2) is subjected to microsampling under a microscope with one single cell each time, placed on an integrated device for rapid pretreatment-ionization-detection to perform online pretreatment, ionization and mass analysis of the sample, and obtain relevant information of a substance in the single cell sample;
Step (5), the mass spectrometry signal data of the substance obtained in steps (3) and (4) are interpreted to obtain biological information.

In some embodiments, in step (5), interpreting multi-cell/single-cell mass spectrometry data is as follows:
(a) mass-to-charge ratio in the multi-cell/single-cell mass spectrometry data is extracted, and the information of a metabolite, lipid, peptide and protein in a single cell is obtained based on the analysis of the mass-to-charge ratio;
(b) the molecular structure information and signal level of the metabolite, lipid, peptide and protein obtained in step (a) are subjected to retrieval and comparison of molecular weight, identification of structure, significance test of signal intensity, calculation of signal variation folds, and identification of a potential significant marker, so as to obtain the heterogeneity between cells and obtain a marker molecule indicating the cell type or cell state, so as to provide accurate analysis for cell molecular diagnosis.

### Definition

SMaRtESI: Sub-Millisecond Scale Resolution Separation electrospray, a method proposed by the present disclosure.

IESI: induced electrospray, wherein a pulsed high voltage is applied to an electrospray needle, and the voltage is applied for a time scale of more than seconds.

ESI: electrospray, wherein a DC high voltage is applied to the tip of an electrospray needle to produce a stable spray without separation effect.

Microelectrophoresis phenomenon refers to a phenomenon that the movement speed of a molecules in SMaRtESI and IESI is different due to the difference in electrical mobility.

Standard solution is a reagent solution with an accurately known concentration.

Timing control: The timing control in the present disclosure is the time sequence between the signal of the pretreament module, the signal of the timing module and the signal of the mass spectrometry module during a single time of sample analysis.

High-precision timing control means that during a single time of sample analysis, the time accuracy of the time sequence between the signal of the pretreatment module, the signal of the timing module and the signal of the mass spectrometry module is at a sub-millisecond level (<1ms).

Low-precision timing control mens that during a single time of sample analysis, the time accuracy of the time sequence between the signal of the pretreatment module, the signal of the timing module and the signal of the mass spectrometry module is at a millisecond level or above (≥1ms).

Heterogeneity between cells means that the same type of cells have differences in intracellular metabolites, proteins, genes, etc. due to differences in the extracellular environment and the stages of cell development and differentiation.

Internal trigger means that the timing control module sends a pulse as a trigger signal.

External trigger means that the mass spectrometer sends a pulse as a trigger signal.

Power trigger means that the rapid pretreatment-ionization device sends a pulse as a trigger signal.

BNC interface (i.e., Bayonet Neill-Concelman connector) is a coaxial signal connection line, which is used for signal transmission between different modules.

Edge trigger means that the level change of the rising or falling segment of the pulse signal is used as the trigger signal.

Pulse width trigger means that the duration interval of the pulse signal is used as the trigger signal.

Slope trigger means that the slope change of the level of the rising or falling segment of the pulse signal is used as the trigger signal.

Duration trigger means that the duration length of the pulse signal is used as the trigger signal.

### Advantages

In the method for sub-millisecond rapid pretreatment of a multi-cell/single-cell sample provided by the present disclosure, "an integrated device for rapid pretreatment-ionization-detection" module is used to achieve sub-millisecond analysis, and conducts rapid pretreatment-ionization mass spectrometry analysis of biological molecules in a multi-cell/single-cell sample. The main idea is to perform timing control of the rapid pretreatment-ionization device and the mass spectrometer, and during the ion injection of mass spectrometer, a rapid pretreatment-ionization source is triggered to perform precise spraying, and any period of the spray is captured in a directional manner, so as to achieve precise capture of the time-sliced micro-electrophoresis process, thereby the mass spectrometer can improve the time resolution of the rapid pretreatment-ionization source, obtain the signal of the spray moment with the highest separation efficiency, maximize the separation efficiency of single cell pretreatment (sub-millisecond separation between metabolites and a complex matrix in a single cell, i.e. pretreatment), achieve ultra-fast online separation of a multi-cell/single-cell sample, improve the separation efficiency of biomolecules in a multi-cell/single-cell sample, and then achieve biological and statistical analysis of biomolecules in a multi-cell/single-cell sample with high coverage, and efficiently obtain the prediction information of cell subtypes and the marker biomolecules related to subtypes. The method can make subtle adjustments to the sampling method of a multi-cell/single-cell sample (subtle adjustments are made by changing the sample to be tested, and the sample to be tested is a multi-cell suspension or homogenate sample or a single-cell sample), and is compatible with the rapid pretreatment mass spectrometry analysis of multi-cell homogenate, cell suspension and other samples.

The multi-cell/single-cell sample online pretreatment mass spectrometry analysis method of the present disclosure realizes ultrafast pretreatment of single-cell samples through timing ultrafast microelectrophoresis separation. The separation speed can reach milliseconds, and the time resolution of detection pretreatment reaches sub-milliseconds. It can realize the separation of multiple biological molecules and interfering substances in a single cell within milliseconds, and realize the separation of substances in the order of molecular weight. It can realize the fine capture during ultrafast microelectrophoresis pretreatment processes in a solution system, a complex matrix, a multi-cell system and a single-cell system, realize efficient online matrix interference removal and ion suppression reduction, and at the same time realize high-coverage detection of multiple biological molecules (including metabolites, lipids, peptides and proteins) with biological functions in a single cell, so as to obtain multi-cell/single-cell sample data with better quality.

### BRIEF DESCRIPTION OF THE DRAWINGS

Figure 1 shows a flow chart of a method for online rapid pretreatment and mass spectrometry analysis of a multi-cell/single-cell sample according to one embodiment of the present disclosure.

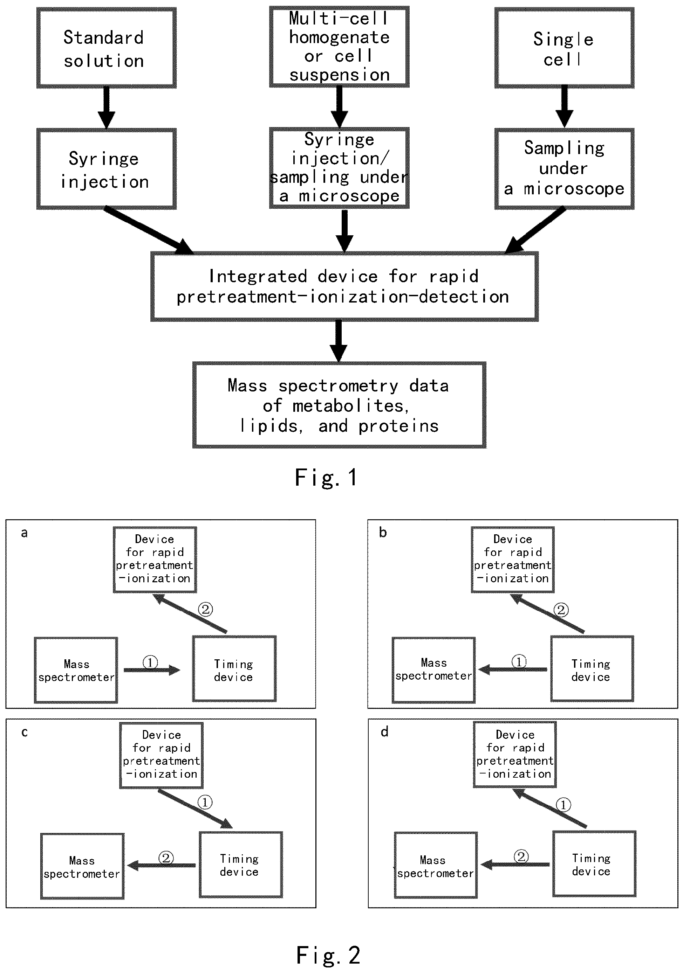

Figure 2 shows a flow chart of four control modes of the integrated device for rapid pretreatment-ionization-detection, where the numbers ① and ② represent the timing of time signals; Figure 2a shows that the mass spectrometer outputs a trigger signal to the timing control device at an adjustable frequency of 1Hz-1M Hz, and the timing control device sends an adjustable 1mV-30V high-level signal to the rapid pretreatment-ionization device; Figure 2b shows that the timing control device monitors the mass spectrometry signal at an adjustable frequency of but not limited to 1 Hz-1 M Hz, and after capturing the mass spectrometry signal, it outputs an adjustable but not limited to 1 mV-30 V high-level signal to the rapid pretreatment-ionization device; Figure 2c shows that during sample pretreatment, the rapid pretreatment-ionization device outputs a trigger signal to the timing control device at an adjustable frequency of but not limited to 1 Hz-1 M Hz, the timing control device sends an adjustable 1mV-30V high-level signal to the mass spectrometer, and the mass spectrometer receives the trigger signal to perform ion injection analysis; Figure 2d shows that the timing control device outputs a trigger signal at an adjustable frequency of but not limited to 1 Hz-1 M Hz, outputs an adjustable 1mV-30V high-level signal to control the rapid pretreatment-ionization device to perform ionization, and triggers the mass spectrometer to collect signals.
Figure 3 shows the sub-millisecond separation and capture of analytes in a mixed solution with different molecular weights (acetylcholine 2µM and rhodamine B 2µM); wherein, (a) shows extracted ion currents of mass spectrometry for acetylcholine and rhodamine B with sub-millisecond time resolution, (b) shows a schematic diagram of the directional capture spray process, (c) shows a mass spectrum captured by the method of the present disclosure with timing control of 0.2, 1.2, and 2.2 ms, and an averaged mass spectrum of random capture.
Figure 4 shows a rapid pretreatment mass spectrum of calmodulin in positive ion mode (sESI-pos) and negative ion mode (sESI-neg).
Figure 5 shows a rapid pretreatment mass spectrum of lipid components, sphingomyelin SM (34:0) and SM (36:1) under positive ion mode.

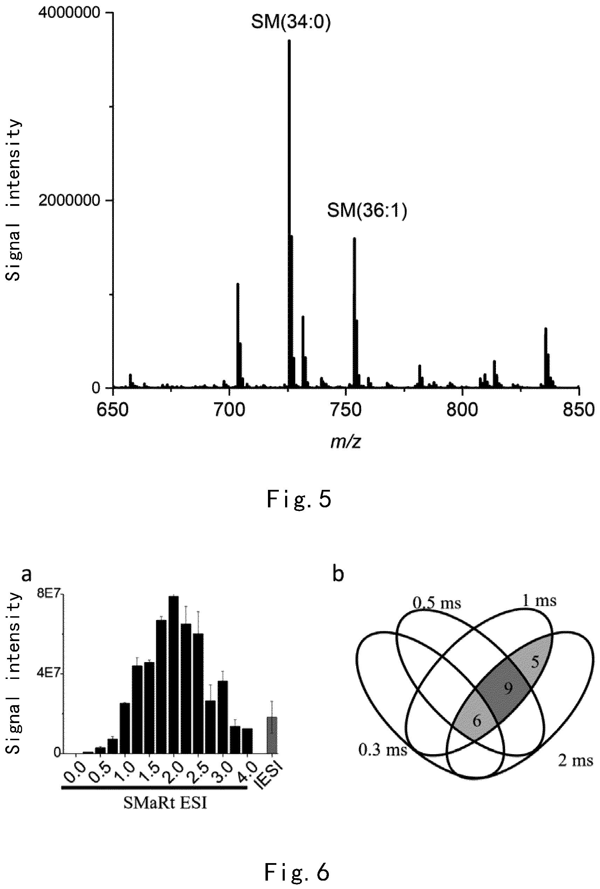

Figure 6 shows the comparison of total ion intensity of the spray of a mixed solution (0.15 µM alanine, 0.15 µM γ-aminobutyric acid, 0.015 µM choline, 0.1 µM creatinine, 0.01 µM proline, 0.01 µM pyroglutamic acid, 0.1 µM creatine, 0.01 µM asparagine, 0.05 µM aspartic acid, 0.005 µM acetylcholine, 0.15 µM spermine, 0.05 µM glutamine, 0.01 µM glutamic acid, 0.01 µM histidine, 0.005 µM carnitine, 0.01 µM arginine, 0.15 µM spermidine, 0.05 µM inosine, 0.05 µM glutathione, 0.05 µM reserpine); (b) shows the distribution of the number of analyte ionizations over the spray time for metabolites captured during spray ionization.
Figure 7 shows a mass spectrum of metabolite analysis of betaine, lysine, arginine, acetylspermidine and glutathione in a living Escherichia coli multi-cell sample.
Figure 8 shows a rapid pretreatment mass spectrum of lipids such as phosphatidylcholine (PC, including PC (d32:0), PC (d34:1), PC (d36:1)) and sphingomyelin (SM, such as SM (d34:1)) in a multi-cell sample, wherein one of the two PC (34:1) peaks is the Na binding peak of PC (34:1) and the other is the K binding peak of PC (34:1).
Figure 9 shows an electrospray ionization effect of calmodulin in a multi-cell sample, and it shows a complete mass spectrum (left) and an enlarged view of the local charge state (right) for the calmodulin in live Escherichia coli by the method of the present disclosure (SMaRtESI), IESI and ESI.
Figure 10 shows an electrospray ionization effect of calmodulin-calcium ion interaction in a multi-cell sample, and it shows a complete mass spectrum (left) and an enlarged view of the local charge state (right) for the calmodulin in live Escherichia coli by the method of the present disclosure (SMaRtESI), IESI and ESI.
Figure 11 shows extracted ion currents of creatine and glutathione in the fine process of ionization microelectrophoresis of an MCF7 single cell, wherein it shows the ion currents of a single cell at 0.2, 1.8, 2.8 and 3.8 ms (left) and metabolic mass spectrum (right).
Figure 12 shows a mass spectrum of cellular metabolites (betanie, leucine, glutamine, arginine, phosphorylcholine, butyrcarnitine, glutathione/GSH) obtained in single cell analysis.
Figure 13 shows an ionization effect of the method of the present disclosure (SMaRtESI, upper panel) and IESI (lower panel) on the lipid component lecithin of a single cell.
Figure 14 shows a complete picture (left) and a local enlarged picture (right) of the ionization effect on all protein components of a single cell.

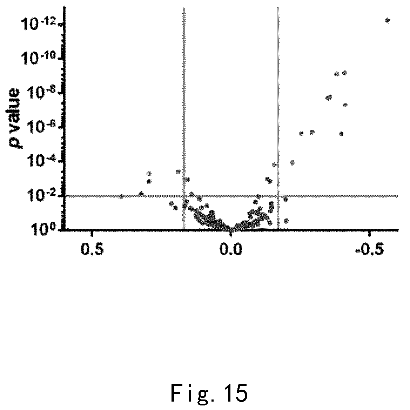

Figure 15 shows a volcano map of metabolite changes in a single cell before and after free radical damage obtained by the method of the present disclosure.

### DETAILED DESCRIPTION OF THE INVENTION

In order to make the purpose, technical solution, and advantage of the present disclosure clear, the present disclosure will be further described in detail below with reference to specific Examples and Figures.

### Example 1. Monitoring of the fine separation process for metabolites of online rapid pretreatment

Step 1, the basic parameters of mass spectrometer were set as follows: a dual-pressure linear ion trap mass spectrometer was seleted from LTQ Velos Pro mass spectrometer (ThermoFisher Scientific, CA, USA) of ThermoFisher Scientific, Inc., and the acquisition parameters thereof were as follows: capillary transmission voltage of 35 V; capillary transmission temperature of 275°C; average spectrum frame number of 1 frame; mass scanning range of 60-900.

Step 2, the signal transmission between the integrated device for rapid pretreatment-ionization-detection and the mass spectrometer was shown in Figure 2, which comprises the following methods: (1) the mass spectrometer firstly sent a signal to the timing device, and then the timing device sent a signal to the rapid pretreatment-ionization device; (2) the timing device firstly sent a signal to the mass spectrometer, and then the timing device sent a signal to the rapid pretreatment-ionization device; (3) the rapid pretreatment-ionization device firstly sent a signal to the timing device, and then the timing device sent a signal to the mass spectrometer, or (4) the timing device firstly sent a signal to the rapid pretreatment-ionization device, and then the timing device sent a signal to the mass spectrometer.

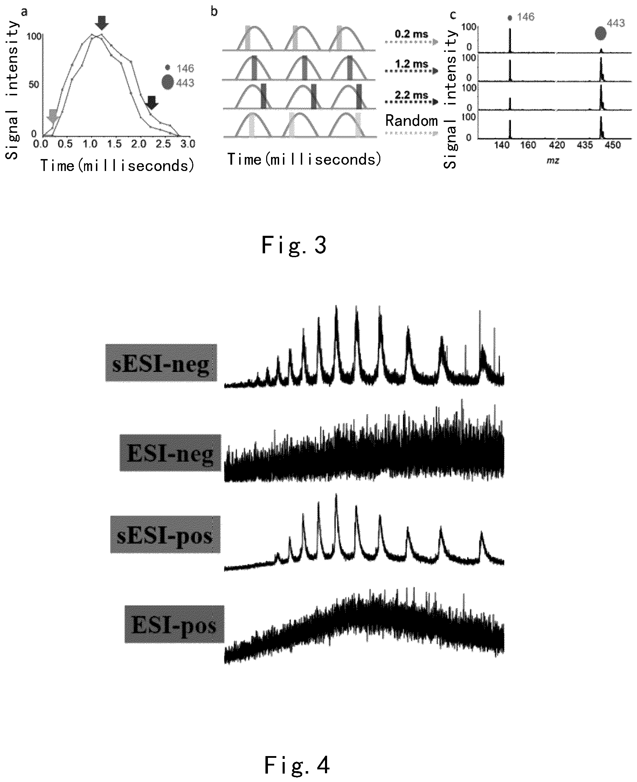

Step 3, a sample containing 2µM acetylcholine and 2µM rhodamine B was injected into a capillary needle through the injector, and then placed into the rapid pretreatment device; an induction pulse voltage consistent with the mass spectrometer ion injection timing was applied to realize the online 200Hz rapid pretreatment, wherein the time points of the timing control were shown in Figure 3b, *i.e.,* 0.2ms, 1.2ms and 2.2ms (Figure 3b).

The extracted ion currents of mass spectrometry of the two molecules (Figure 3a) showed that there is a sub-millisecond phase difference between the ion currents of acetylcholine with a smaller molecular weight and rhodamine B with a larger molecular weight, and acetylcholine with a smaller molecular weight tends to ionize in the early stage of spraying, while rhodamine B tends to ionize in the late stage of spraying (in the ion current diagram, the peak time of the rhodamine B signal lags behind the peak time of the acetylcholine signal). As seen from the specific spray mass spectrum (Figure 3c), at 0.2 ms in the initial stage of spraying, the relative abundance of acetylcholine with a smaller molecular weight is the highest in the mass spectrum, and as the spraying process continues, the signal of rhodamine B with a larger molecular weight gradually increases. When the spraying reaches 1.2 ms, the signal of rhodamine B is equivalent to that of acetylcholine, and when the spraying reaches 2.2 ms, the relative abundance of rhodamine B exceeds that of acetylcholine. This further confirms that there is a certain degree of microelectrophoresis in the early stage of spraying, which can achieve ultrafast separation and pretreatment of metabolites with different electrical mobilities, and achieve highly sensitive analysis of metabolites; the advantages over a random separation process are in that it can achieve accurate mass spectrometry acquisition for the pretreatment of metabolites by regulating the spray process, and obtain the mass spectrometry signal with the best rapid pretreatment effect.

### Example 2. Efficient analysis of proteins, lipids, metabolites, etc. in standard solutions by online rapid pretreatment

Step 1, the basic parameters of mass spectrometer were set as follows: a dual-pressure linear ion trap mass spectrometer was seleted from LTQ Velos Pro mass spectrometer (ThermoFisher Scientific, CA, USA) of ThermoFisher Scientific, Inc., and the acquisition parameters thereof were as follows: capillary transmission voltage of 35 V; capillary transmission temperature of 275°C; average spectrum frame number of 1 frame; mass scanning range of 60-900. The dual-pressure linear ion trap mass spectrometer was used for high-precision timing control in timing control.

An orbital trap mass spectrometer was selected from Exactive Plus mass spectrometer (ThermoFisher Scientific, CA, USA), and the acquisition parameters thereof were as follows: S-Lens RF level of 50%; capillary transmission temperature of 275°C; average spectrum frame number of 1 frame; mass resolution of 70000; mass scanning range of 60-900; AGC threshold of 1e6. The orbital trap mass spectrometer could be used for low-precision timing control in timing control.

Step 2, the signal transmission between the integrated device for rapid pretreatment-ionization-detection and the mass spectrometer was the same as that in step 2 of Example 1.

Step 3, a cell lysate with high expression of calmodulin and green fluorescent protein was injected into a capillary and placed in the rapid pretreatment device; the rapid pretreatment device applied a pulsed high voltage of 10kV 500Hz to the capillary for online rapid pretreatment, and the timing device controlled the mass spectrometer to collect the mass spectrum.

The obtained mass spectrum was shown in Figure 4. For samples without pretreatment (*i.e.,* ionization via conventional electrospray), the signals were designated as negative ion mode "ESI-neg" and positive ion mode "ESI-pos", and the protein signal-to-noise ratios thereof in mass spectrum were extremely poor, and protein signals could not be identified; for samples with pretreatment by the method of the present disclosure, the signals were shown in negative ion mode "sESI-neg" and positive ion mode "sESI-pos", wherein the protein solutions presented clear mass spectrometry signals with extremely high signal-to-noise ratios.

Step 4, a solution containing 200 ppm of lipids such as sphingomyelin SM (34:0) and SM (36:1) was infused into the capillary, wherein the number of pulses was 3, the waveform was adjusted to a sine wave, and the other parameters were the same as those in Example 1, and then a timing sampling was performed by mass spectrometry.

The results were shown in Figure 5, which showed that after rapid pretreatment of the sample, the sphingomyelin in the solution could be efficiently ionized and detected by mass spectrometry, and a sphingomyelin signal with a very high signal-to-noise ratio was obtained.

Step 5: A mixed solution containing 0.15 µM alanine, 0.15 µM γ-aminobutyric acid, 0.015 µM choline, 0.1 µM creatinine, 0.01 µM proline, 0.01 µM pyroglutamic acid, 0.1 µM creatine, 0.01 µM asparagine, 0.05 µM aspartic acid, 0.005 µM acetylcholine, 0.15 µM spermine, 0.05 µM glutamine, 0.01 µM glutamic acid, 0.01 µM histidine, 0.005 µM carnitine, 0.01 µM arginine, 0.15 µM spermidine, 0.05 µM inosine, 0.05 µM glutathione, and 0.05 µM reserpine (used to test the general applicability of the method of the present disclosure for enhancing metabolite signals) was infused into the capillary, wherein the waveform was adjusted to a sine wave, and the other parameters were consistent with those in Example 1, and timing sampling was performed by mass spectrometry.

The method of the present disclosure can achieve rapid pretreatment, reduce the interference of metabolites, and improve the response of metabolites. The sum of the total ion intensity of the metabolite signal (Figure 6) shows a tendency of first rising and then falling during the spray process, and the signal intensity reaches the highest value in the middle of the spray. In addition, during the spray process, the number of ionization of metabolites also changes regularly with the spray cycle (Figure 6). In the early stage of the spray, only 6 ions are ionized, and as the spray process progresses, 16 metabolites are ionized in the middle of the spray. At the end of the spray, all substances are mixed together and ionized at the same time, and all metabolites can be ionized. Therefore, due to the reduction of ion suppression, the signal of the analytical method of the present disclosure will be further enhanced. Compared with conventional analytical methods such as electrospray, it realizes sub-millisecond separation, can obtain higher metabolite signals, and realize more sensitive metabolite detection.

### Example 3. Efficient analysis of proteins, lipids, metabolites, etc. in a multi-cell sample by online rapid pretreatment

Step 1, the basic parameters of mass spectrometer were set as follows: a dual-pressure linear ion trap mass spectrometer was seleted from LTQ Velos Pro mass spectrometer (ThermoFisher Scientific, CA, USA) of ThermoFisher Scientific, Inc., and the acquisition parameters thereof were as follows: capillary transmission voltage of 35 V; capillary transmission temperature of 275°C; average spectrum frame number of 1 frame; mass scanning range of 60-900.

Step 2, the signal transmission between the integrated device for rapid pretreatment-ionization-detection and the mass spectrometer was the same as that in step 2 of Example 1.

Step 3, an *Escherichia coli* suspension was infused into the capillary, wherein the waveform was adjusted to a sine wave, and the other parameters were the same as those in Example 1.

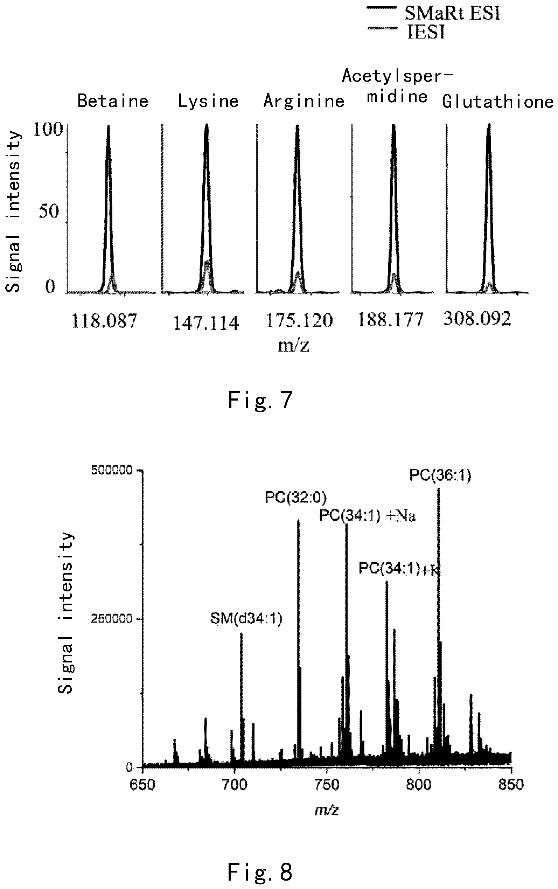

In the method of the present disclosure, a rapid release of metabolites in cells with cell walls was achieved via a pulse voltage (whereas a direct voltage is used in the prior art, which cannot quickly release metabolites of cells with cell walls), spray moments with the best separation effect of intracellular metabolites and matrix were captured through directional rapid pretreatment, and the signal intensity of metabolites in living cells was higher; as shown in Figure 7 (wherein the gray line represents the test result of IESI, the black line represents the test result of the method of the present disclosure, and the ordinate represents the signal intensity), for metabolites of different molecular weights in cells such as betaine (mz=118.087), lysine (lysine, mz=147.114), arginine (argnine, mz=175.120), acetylspermidine (mz=188.177) and glutathione (mz=308.092), the signal intensities were improved by 4-fold by the method of the present disclosure (Figure 7).

Step 4, a multi-cell suspension was infused into the capillary, wherein the waveform was adjusted to a sine wave, and the other parameters were the same as those in Example 1. A rapid pretreatment of the inner membrane components of *Escherichia coli* was achieved by the method of the present disclosure, wherein the lipid components thereof were quickly released from the cell membrane and ionized, and the ionization signal was accurately captured. The obtained signal intensity for cell lipid was higher and the signal-to-noise ratio was better (Figure 8), and the mass spectrometry signals of lipids such as phosphatidylcholine (PC, including PC (d32:0), PC (d34:1), PC (d36:1)) and sphingomyelin (SM, such as SM (d34:1)) of the multi-cell sample can be obtained.

Step 5, an *Escherichia coli* suspension was infused into the capillary, wherein the waveform was adjusted to a sine wave, and the other parameters were the same as those in Example 1.

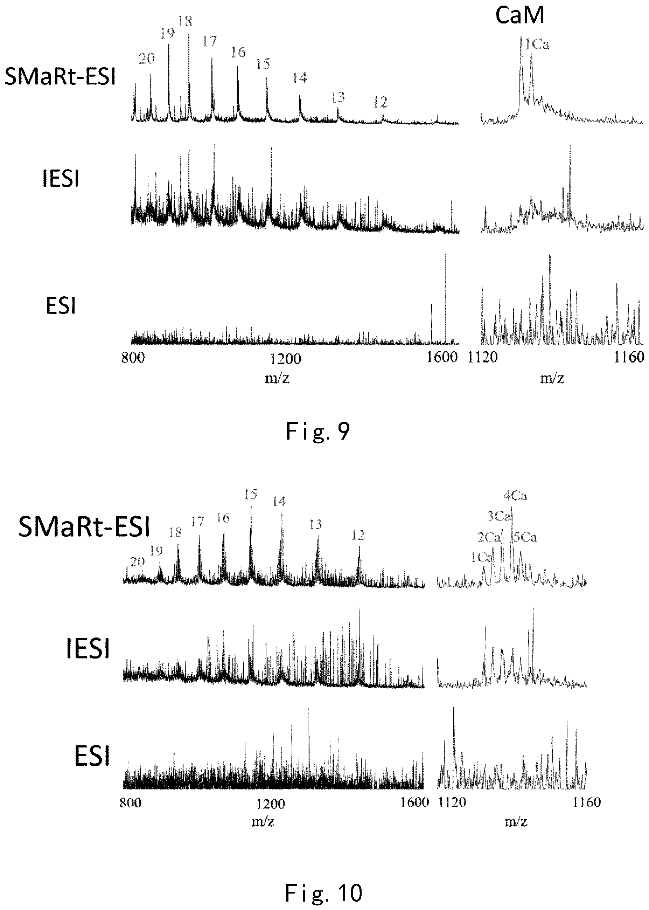

In the method of the present disclosure, an ultra-fast release of intracellular proteins was achieved, wherein the separation of analytes from the matrix congested environment was achieved within milliseconds, and matrix interference was reduced. In the method of the present disclosure (SMaRtESI), calmodulin in living cells was detected with a high signal ratio (Figure 9), and a clearer distribution of charge state of calmodulin was obtained. In the enlarged view of the local charge state (Figure 9), it was observed that in bacteria under original growth conditions, calmodulin existed in the form of free CaM and CaM+1Ca bound to one calcium ion in living cells. In other methods (IESI and ESI), the calmodulin signal was masked by a large number of interfering signals, wherein the signal was relatively poor, and the protein was difficult to be identified (Figure 9). In the enlarged view of the local charge state, the existence form of protein was difficult to be distinguished, and there was strong background interference.

Step 6, the Escherichia coli suspension was mixed with 50µM calcium ions, and infused into the capillary, wherein the waveform was adjusted to a sine wave, and the other parameters were the same as those in Example 1.

In the method of the present disclosure, the interaction of calmodulin and calcium ions in living cells can be detected with a high signal ratio (Figure 10). In the enlarged view of the local charge state, after stimulation of cells by calcium ions, it is observed that calmodulin is converted from a free state to a binding state in living cells, and there are multiple binding forms with calcium ions, forming the state of CaM+1Ca (*i.e.,* CaM plus 1 Ca), CaM+2Ca, CaM+3Ca and CaM+4Ca, wherein the binding state of CaM+4Ca is the main one, indicating that the method of the present disclosure can achieve the detection of intracellular proteins and their binding states with metal ions. The spectral signals of other methods (IESI and ESI) are relatively poor, and proteins are difficult to be identified. In the enlarged view of the local charge state, the existence form of proteins is difficult to be distinguished, and there is strong background interference. Therefore, the method of the present disclosure can achieve sub-millisecond separation of proteins and obtain protein spectra with higher signal intensity and higher signal-to-noise ratio.

### Example 4. Efficient analysis of proteins, lipids, metabolites, etc. in a single cell sample by online rapid pretreatment

Step 1, the basic parameters of mass spectrometer were set as follows: a dual-pressure linear ion trap mass spectrometer was seleted from LTQ Velos Pro mass spectrometer (ThermoFisher Scientific, CA, USA) of ThermoFisher Scientific, Inc., and the acquisition parameters thereof were as follows: capillary transmission voltage 35 V; capillary transmission temperature 275°C; average spectrum frame number, 1 frame; mass scanning range, 60-900.

Step 2, the signal transmission between the integrated device for rapid pretreatment-ionization-detection and the mass spectrometer was the same as that in step 2 of Example 1.

Step 3, cells were taken from an incubator, and the culture medium was replaced with a PBS solution which was perfomed by aspirating the culture medium with a pipette and replacing it with PBS solution; cell were washed with PBS for 3 times, placed in a pure PBS solution, and then the culture dish containing the cells was placed under an optical microscope for the rapid location of cells via a 4x magnification objective; after a clear cell outline was seen in the microscope eyepiece, the microscope objective was changed to a 20x magnification objective to accurately locate the cells.

Step 4, a single-cell sampling electrode was infused with an electrode liquid containing 185 mM NH₄HCO₃ and 80 mM NH₄Cl, fixed on a single-cell three-axis micromanipulator, extended into the culture dish through a rocker arm; the location of the sampling electrode was found in the eyepiece of the microscope by moving a cantilever; the sampling electrode needle tip was manually adjusted so that it hovered just above the single cell; the sampling electrode was inserted into the cell by a Z-axis spiral fine-tuning knob, and then the pipette was quickly removed from the cell and analyzed by mass spectrometer.

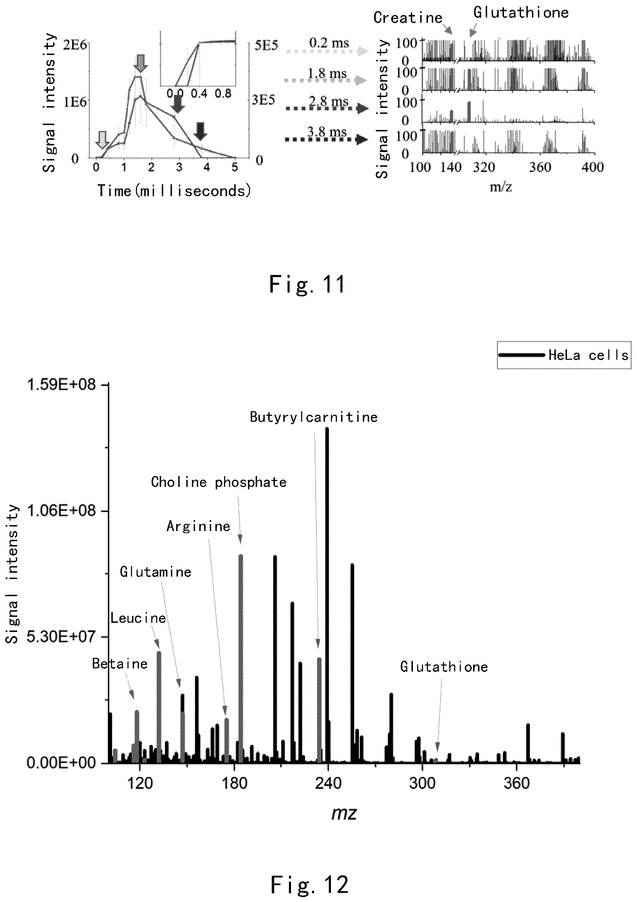

In the method of the present disclosure, an ultrafast separation of metabolites in a single-cell environment was achieved. As shown in Figure 11, there is a certain degree of separation effect for creatine and glutathione in the extracted ion current diagram of metabolites. As shown in the mass spectrum (Figure 11), metabolites with lower molecular weight, such as creatine, have stronger metabolite mass spectrometry signals at 0.2 ms in the single-cell electrospray process, while metabolites with smaller molecular weight, such as glutathione, are not detected at this moment. At the 0.4 ms of spray moment, the signal of glutathione gradually appears; at the end of the spray, the signals of glutathione and creatine are detected. The above results show that, in the method of the present disclosure, millisecond-level rapid pretreatment of samples at a single-cell level is achieved, and the resolution of metabolite ionization process is improved into sub-millisecond level in single-cell analysis.

Step 5, the timing delay time was fixed to obtain a single-cell spray moment with the best rapid pretreatment effect.

Figure 12 is the mass spectrum of cell metabolites (betanie, leucine, glutamine, arginine, phosphorylcholine, butyrcarnitine, glutathione/GSH) obtained by the method of the present disclosure in single-cell analysis.

As shown in Figure 12, in the method of the present disclosure, 73 metabolites are detected in a single cell, which is much higher than the 37 metabolites measured by conventional induction electrospray mass spectrometry. Most metabolites are detected at a single cell level due to the application of the new system, which proves that the system can effectively improve the sensitivity of metabolite detection and obtain higher metabolite coverage in single cell metabolite analysis.

Step 6: the intracellular substances in a single cell were sampled under a microscope, and subjected to further rapid pretreatment of separation using the method of the present disclosure.

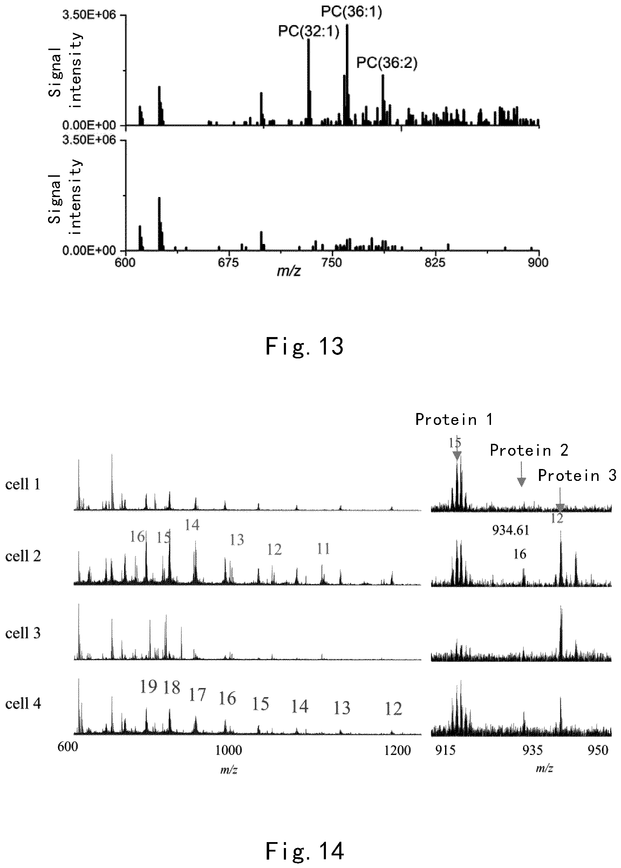

In the method of the present disclosure, a mass spectrum with high signal-to-noise ratio was obtained for lipids and proteins in a single cell. As shown in Figure 13, for lecithin such as PC (32:1), PC (36:1), PC (36:2) and other substances in a single cell, the signal intensity obtained by the method of the present disclosure was high (upper panel), while the signal obtained by the conventional induction electrospray IESI method as a control group was submerged in noise (lower panel) due to insufficient pretreatment efficiency. For proteins in a single cell, the method of the present disclosure could pre-treat and separate the inorganic salt interfering components in the cell, realize protein ionization and detection within milliseconds (Figure 14), and realize mass spectrometry detection of proteins in a single cell.

### Example 5. Subtype heterogeneity analysis of free radical damage in multi-cell/single-cell by online rapid pretreatment

Step 1, the basic parameters of mass spectrometer were set as follows: a dual-pressure linear ion trap mass spectrometer was seleted from LTQ Velos Pro mass spectrometer (ThermoFisher Scientific, CA, USA) of ThermoFisher Scientific, Inc., and the acquisition parameters thereof were as follows: capillary transmission voltage of 35 V; capillary transmission temperature of 275°C; average spectrum frame number of 1 frame; mass scanning range of 60-900.

Step 2, the signal transmission between the integrated device for rapid pretreatment-ionization-detection and the mass spectrometer was the same as that in step 2 of Example 1.

Step 3, cells were incubated with 0.1% hydrogen peroxide, and cells incubated without hydrogen peroxide were used as a control group. After 1 hour, cells were taken from the incubator, and the culture medium was replaced with PBS solution which was perfomed by aspirating the culture medium with a pipette and replacing it with PBS solution; cell were washed with PBS for 3 times, placed in a pure PBS solution, and then the culture dish containing the cells was placed under an optical microscope for the rapid location of cells via a 4x magnification objective; after a clear cell outline was seen in the microscope eyepiece, the microscope objective was changed to a 20x magnification objective to accurately locate the cells.

Step 4, a single-cell sampling electrode was infused with an electrode liquid containing 185 mM NH₄HCO₃ and 80 mM NH₄Cl, fixed on a single-cell three-axis micromanipulator, extended into the culture dish through a rocker arm; the location of the sampling electrode was found in the eyepiece of the microscope by moving a cantilever; the sampling electrode needle tip was manually adjusted so that it hovered just above the single cell; the sampling electrode was inserted into the cell by a Z-axis spiral fine-tuning knob, and then the pipette was quickly removed from the cell and analyzed by mass spectrometer.

Step 5: Rapid pretreatment and mass spectrometry detection of intracellular substances of a single cell were performed and metabolite data were extracted. The fold of metabolic change and p-value were compared and calculated between cells before and after free radical damage. After 0.1% hydrogen peroxide treatment, the signal levels of 8 metabolites were increased by 1.2 folds, of which 6 showed a significant difference (p<0.05); the signal levels of 12 metabolites were decreased by 0.8 folds, of which 10 showed a significant difference (p<0.05). This showed that the heterogeneity of metabolites in cells before and after free radical damage was completely different with significant up- and down-regulations, which verified that, in the present disclosure, sub-millisecond separation of metabolites in a single cell was achieved and mass spectrometry signals of metabolites with higher coverage in a single cell were obtained.

The specific Examples described above further illustrate the purpose, technical solutions and beneficial effects of the present disclosure in details, but they should not be construed as limiting the scope of the disclosure. It should be noted that any modifications, equivalent replacements and improvements made by those skilled in the art within the spirit and scope of the disclosure should be included within the scope of the present disclosure.

## Claims

1. An integrated device for rapid pretreatment-ionization-detection, wherein the device comprises:
(a) a fixing device for fixing a sample to be treated, wherein the fixing device comprises a support frame for supporting a sample tube and a support frame for supporting an electrode for pretreatment and ionization; the support frame for supporting the sample tube is configured to fix the sample by a snap-on or flat-lay contact; the electrode for pretreatment and ionization is a non-contact electrode or a contact electrode, to apply a voltage to the sample;
(b) a rapid pretreatment-ionization device, wherein the rapid pretreatment-ionization device comprises: a voltage output module for generating an AC voltage; a boost module for boosting and amplifying the voltage to achieve an adjustable pulse voltage of 1mV-100 kV; a delay module for setting a delay time for the voltage output; a trigger module for receiving a pulse voltage trigger;
(c) a mass spectrometer; and
(d) a timing control device for controlling the mass spectrometer and the rapid pretreatment-ionization device to collect a signal, wherein the timing control is any of the following:
(1) the mass spectrometer outputs a trigger signal to the timing control device at an adjustable frequency of 1Hz-1M Hz, and the timing control device sends an adjustable 1mV-30V high-level signal to the rapid pretreatment-ionization device;
(2) during the sample pretreatment, the rapid pretreatment-ionization device outputs a trigger signal to the timing control device at an adjustable frequency of 1Hz-1M Hz, the timing control device sends an adjustable 1mV-30V high-level signal to the mass spectrometer, and the mass spectrometer receives the trigger signal to perform an ion injection analysis;
(3) the timing control device outputs a trigger signal at an adjustable frequency of 1Hz-1M Hz, outputs an adjustable 1mV-30V high-level signal to control the rapid pretreatment-ionization device to perform ionization, and triggers the mass spectrometer to collect the signal;
(4) the timing control device monitors the mass spectrometry signal at an adjustable frequency of 1Hz-1M Hz, and after capturing the mass spectrometry signal, it outputs an adjustable 1mV-30V high-level signal to the rapid pretreatment-ionization device;
wherein the detection of a positive ion signal and a negative ion signal is achieved by switching a detection mode of the mass spectrometer.

2. The integrated device for rapid pretreatment-ionization-detection of claim 1, wherein the AC voltage output by the voltage output module is a sine wave, a pulse wave, or a square wave.

3. The integrated device for rapid pretreatment-ionization-detection of claim 1, wherein the adjustable pulse voltage achieved by the boost module is in a range of 1m Hz-100 kHz, with a pulse cycle output number of 1-100k cycles.

4. The integrated device for rapid pretreatment-ionization-detection of claim 1, wherein the delay time is 1ns-100ks.

5. The integrated device for rapid pretreatment-ionization-detection of claim 1, wherein the pulse voltage trigger received by the trigger module is positive polarity 1mV-5V or negative polarity 1mV-5V to realize an ion signal detection in a positive ion mode and a negative ion mode.

6. The integrated device for rapid pretreatment-ionization-detection of claim 1, wherein the pulse voltage trigger received by the trigger module is in a mode selected from edge trigger, pulse width trigger, slope trigger and duration trigger.

7. The integrated device for rapid pretreatment-ionization-detection of claim 1, wherein the pulse voltage trigger received by the trigger module is in an AC cycle of the rapid pretreatment, with a speed consistent with pulse width of a pulse cycle, and an AC high voltage is converted to a DC high voltage by connecting a capacitor and a high-voltage rectifier in front of the electrode.

8. The integrated device for rapid pretreatment-ionization-detection of claim 1, wherein the mass spectrometer is selected from a magnetic mass spectrometer, a quadrupole mass spectrometer, an ion trap mass spectrometer, an orbital trap mass spectrometer, a time-of-flight mass spectrometer, and a Fourier transform ion cyclotron resonance mass spectrometer.

9. A method for metabolic mass spectrometry analysis of a multi-cell or a single-cell sample, wherein the method comprises the following steps:
Step (1), a standard solution is filterd to remove fine particles for later use; as for a multi-cell sample such as a tissue block or a bacterial liquid, the sample is crushed to form a homogenate, or the sample is dispersed to prepare a cell suspension; as for an experimentally synthesized sample or an environmental sample containing a complex matrix, such as an animal or plant sample, a soil sample, a river water sample and a sediment sample, the sample is treated to obtain a solution suspension or a supernatant by centrifugation; as for a sample containing a single cell, the sample is subjected to single cell extraction, culture or dispersion to obtain a single cell suspension or a single cell monolayer slide;
Step (2), the homogenate or cell suspension obtained in step (1) is subject to direct sampling with syringe extraction or sampling under a microscope, with a sampling volume of, for example, 0.001 pL-10 mL, loading on the integrated device for rapid pretreatment-ionization-detection of any one of claims 1-8 to perform an online pretreatment, ionization and mass analysis of the sample, so as to obtain relevant information of a substance in the sample; or the single-cell sample in step (1) is subject to microsampling under a microscope with one single cell each time, loading on the integrated device for rapid pretreatment-ionization-detection of any one of claims 1-8 to perform an online pretreatment, ionization and mass analysis of the sample, so as to obtain relevant information of a substance in the single-cell sample;
Step (3), mass spectrometry data of the substance obtained in step (2) is interpreted to obtain biological information.

10. The method for metabolic mass spectrometry analysis of a multi-cell or a single-cell sample according to claim 9, wherein step (3) of interpreting a multi-cell or a single-cell mass spectrometry data comprises:
(a) a mass-to-charge ratio in the multi-cell or single-cell mass spectrometry data is extracted, and information of a metabolite, lipid, peptide and protein in the single cell is obtained based on an analysis of the mass-to-charge ratio;
(b) a molecular structure information and a signal level of the metabolite, lipid, peptide and protein obtained in step (a) are subjected to retrieval and comparison of a molecular weight, a structure identification, a significance test of signal intensity, a calculation of signal variation, and an identification of a potential significant marker, so as to obtain heterogeneity between cells and obtain a marker molecule indicating a cell type or cell state, and thus provide an accurate analysis for a cell molecular diagnosis.
